# EUROPÄISCHE PATENTSCHRIFT

(11) **EP 1 235 813 B1**
(45) Veröffentlichungstag und Bekanntmachung des Hinweises auf die Patenterteilung: **06.04.2005**
(21) Anmeldenummer: 00971425.4
(22) Anmeldetag: 06.11.2000
(51) Int. Cl.: C07D 239/52, A01N 43/54

(54) **HYDROXAMSÄUREDERIVATE**
HYDROXAMIC ACID DERIVATIVES
DERIVES D'ACIDE HYDROXAMIQUE

(30) Priorität: 17.11.1999 DE 19955130
(43) Veröffentlichungstag der Anmeldung: 04.09.2002
(73) Patentinhaber: Bayer CropScience AG, 40789 Monheim (DE)
(72) Erfinder: KRÜGER, Bernd-Wieland, 51467 Bergisch Gladbach (DE); GAYER, Herbert, 40789 Monheim (DE); GERDES, Peter, 52080 Aachen (DE); MAURER, Fritz, 40789 Monheim (DE); HEINEMANN, Ulrich, 42799 Leichlingen (DE); VAUPEL, Martin, 42799 Leichlingen (DE); MAULER-MACHNIK, Astrid, 42799 Leichlingen (DE); WACHENDORFF-NEUMANN, Ulrike, 56566 Neuwied (DE); HÄNSSLER, Gerd, 51381 Leverkusen (DE); KUCK, Karl-Heinz, 40764 Langenfeld (DE)
(74) Vertreter: Weiss, Wolfgang, Dipl.-Chem. Dr.
(86) Internationale Anmeldenummer: PCT/EP2000/010918
(87) Internationale Veröffentlichungsnummer: WO 2001/036393

(56) Entgegenhaltungen:
- WO-A-00/10970
- WO-A-00/71504
- WO-A-00/78732
- WO-A-00/78733
- WO-A-95/20570
- WO-A-98/21189

## Beschreibung

Die Erfindung betrifft neue Hydroxamsäurederivate mehrere Verfahren zu ihrer Herstellung und ihre Verwendung als Schädlingsbekämpfungsmittel.

Bestimmte Hydroxamsäurederivate mit ähnlichem Substitutionsmuster, sowie deren fungizide Wirkung sind bereits bekannt geworden (vergleiche z. B. WO 95-20570)). Die Wirkung dieser vorbekannten Verbindungen ist jedoch insbesondere bei niedrigen Aufwandmengen und Konzentrationen nicht in allen Anwendungsgebieten völlig zufriedenstellend.

Es wurden nun die neuen Hydroxamsäurederivate der allgemeinen Formel (I) gefunden, 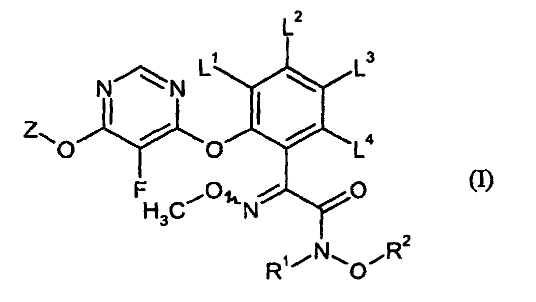 in welcher
- Z: für jeweils gegebenenfalls einfach bis zweifach durch Halogen, Alkyl, oder Hydroxy substituiertes Cycloalkyl mit 3 bis 7 Kohlenstoffatomen;
für gegebenenfalls durch Alkyl mit 1 bis 4 Kohlenstoffatomen substituiertes Heterocyclyl mit 3 bis 7 Ringgliedern;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino,
Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 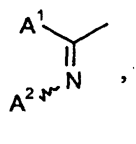 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht, sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen,
- L¹, L², L³ und L⁴: gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen,
- R¹: für Wasserstoff oder Alkyl steht und
- R²: für Wasserstoff, Alkyl, Alkylcarbonyl, Alkoxycarbonyl oder Arylcarbonyl steht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie beispielsweise in Alkoxy, Alkylthio oder Alkylamino, jeweils geradkettig oder verzweigt. Bevorzugt sind, wenn nicht anders angegeben, Kohlenwasserstoffketten mit 1 bis 6 Kohlenstoffatomen.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl, die gegebenenfalls auch mit weiteren aliphatischen oder heterocyclischen Ringen kondensiert sein können.So steht Aryl beispielsweise auch für Tetralinyl, Indolyl, oder Benzofuranyl, wobei die Anknüpfung aber am Phenylteil erfolgt.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält der Ring mehrere Sauerstoffatome, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Ein polycyclisches Ringsystem kann mit einem heterocyclischen Ring oder einem ankondensierten carbocyclischen Ring verknüpft sein. Das so beschriebene Heterocyclyl kann auch einfach oder mehrfach substituiert sein, vorzugsweise durch Methyl, Ethyl, Halogen oder Chlor. Bevorzugt sind mono- oder bicyclische Ringsysteme, insbesondere mono- oder bicyclische Ringsysteme.

Halogenalkoxy steht für teilweise oder vollständig halogeniertes Alkoxy. Bei mehrfach halogeniertem Halogenalkoxy können die Halogenatome gleich oder verschieden sein. Bevorzugte Halogenatome sind Fluor und insbesondere Chlor. Trägt das Halogenalkoxy noch weitere Substituenten, reduziert sich die maximal mögliche Zahl der Halogenatome auf die verschiedenen freien Valenzen.

Halogenalkyl steht für teilweise oder vollständig halogeniertes Alkyl. Bei mehrfach halogeniertem Halogenalkyl können die Halogenatome gleich oder verschieden sein. Bevorzugte Halogenatome sind Fluor und Chlor, insbesondere Fluor. Trägt das Halogenalkyl noch weitere Substituenten, reduziert sich die maximal mögliche Zahl der Halogenatome auf die verbleibenden freien Valenzen.

Weiterhin wurde gefunden, dass man die neuen Hydroxamsäurederivate der allgemeinen Formel (I) erhält, wenn man
a) Carbonsäurederivate der Formel (II), 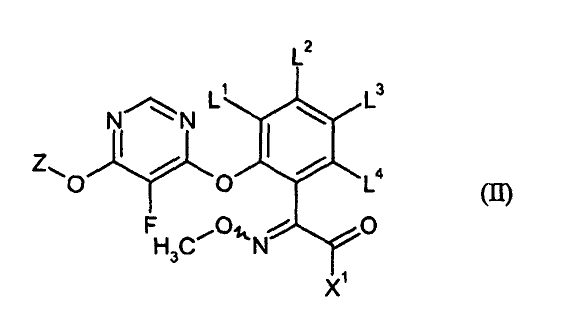 in welcher
   - L¹, L², L³, L⁴ und Z: die oben angegebenen Bedeutungen haben und
   - X¹: für Halogen steht,
   mit einem substituierten oder unsubstituierten Hydroxylaminderivat der allgemeinen Formel (III), 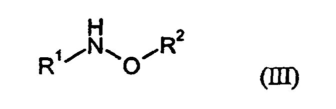 in welcher
   - R¹ und R²: die oben angegebenen Bedeutungen haben,
   gegebenenfalls in Gegenwart eines Verdünnungsmittels und gegebenenfalls in Gegenwart eines Säureakzeptors, umsetzt, oder wenn man
b) Verbindungen der allgemeinen Formel (I) mit R² in seiner Bedeutung als Wasserstoff
   mit einer Halogenverbindung der allgemeinen Formel (IV),

   X²-R^{2'} (IV)

   in welcher
   - R^{2'}: für Alkyl, Alkylcarbonyl, Alkoxycarbonyl oder Arylcarbonyl steht, und
   - X²: für Halogen steht,
   gegebenenfalls in Gegenwart eines Verdünnungsmittels, gegebenenfalls in Gegenwart eines Säureakzeptors, umsetzt.

Schließlich wurde gefunden, dass die neuen Hydroxamsäurederivate der allgemeinen Formel (I) eine sehr starke sehr starke Wirkung gegen Pflanzenschädlinge zeigen.

Die erfindungsgemäßen Verbindungen können gegebenenfalls als Mischungen verschiedener möglicher isomerer Formen, insbesondere von Stereoisomeren, wie z. B. E- und Z-, vorliegen. Es werden sowohl die E- als auch die Z-Isomeren, wie auch beliebige Mischungen dieser Isomeren, beansprucht.

Gegenstand der Erfindung sind vorzugsweise Verbindungen der Formel (I), in welcher
- Z: für jeweils gegebenenfalls einfach bis zweifach durch Fluor, Chlor, Methyl, Ethyl oder Hydroxy substituiertes Cyclopentyl oder Cyclohexyl;
für gegebenenfalls durch Methyl oder Ethyl substituiertes Thienyl, Pyridyl oder Furyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl,
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), Hydroxymethyl, Hydroxyethyl, 3-Oxobutyl, Methoxymethyl, Dimethoxymethyl,
Methoxy, Ethoxy, n- oder i-Propoxy, Methoxymethyl, Ethoxymethyl,
Methylthio, Ethylthio, n- oder i-Propylthio, Methylsulfinyl, Ethylsulfinyl, Methylsulfonyl oder Ethylsulfonyl, Methylthiomethyl, Ethylthiomethyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy oder eine Gruppierung 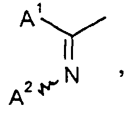 wobei
A¹ für Wasserstoff, Methyl oder Hydroxy steht und
A² für Hydroxy, Methoxy, Ethoxy, Amino, Methylamino, Phenyl, Benzyl oder Hydroxyethyl steht, sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl,
- L¹, L², L³ und L⁴: gleich oder verschieden sind und unabhängig voneinander jeweils für Wasserstoff, Methyl, Ethyl, n- oder i-Propyl, n-, i-, s- oder t-Butyl stehen,
- R¹: für Wasserstoff oder Alkyl steht,
- R²: für Wasserstoff, Alkyl, Alkylcarbonyl, Alkoxycarbonyl oder Arylcarbonyl steht.

In einer ganz besonders bevorzugten Gruppe von Verbindungen steht Z für gegebenenfalls substituiertes Phenyl.

Besonders bevorzugt sind Verbindungen der Formel (I), in denen R¹ für Wasserstoff, insbesondere für Methyl steht.

Besonders bevorzugt sind Verbindungen der Formel (I), in denen Z für gegebenenfalls substituiertes Phenyl steht, wobei die Substituenten vorzugsweise aus der nachstehenden Aufzählung ausgewählt sind: Halogen, Cyano, jeweils geradkettiges oder verzweigtes Alkyl, Alkylthio, Alkylthioalkyl, Halogenalkyl, Halogenthioalkyl.

In einer weiteren ganz besonders bevorzugten Gruppe von Verbindungen stehen
- L¹, L² und L³: für Wasserstoff und
- L⁴: für Wasserstoff oder für Methyl.

Die Erfindung betrifft besonders bevorzugt Verbindungen der Formel (I), in welcher
- Z: für jeweils gegebenenfalls einfach bis dreifach, gleich oder verschieden substituiertes Phenyl steht, wobei die Substituenten vorzugsweise aus der nachstehenden Aufzählung ausgewählt sind: Fluor, Chlor, C₁-C₄-Alkyl oder Cyano,
- L¹, L², L³ und L⁴: für Wasserstoff stehen,
- R¹: für C₁-C₄-Alkyl steht, und
- R²: für Wasserstoff oder Methyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination der Reste, beliebig auch durch Restedefinitionen anderer Vorzugsbereiche ersetzt.

Die zur Durchführung des erfindungsgemäßen Verfahrens a) als Ausgangsstoffe benötigten Carbonsäurederivate sind durch die Formel (II) allgemein definiert. In dieser Formel (II) haben L¹, L², L³, L⁴ und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für L¹, L², L³, L⁴ und Z angegeben wurden. X¹ steht für Halogen, vorzugsweise für Chlor.

Die Ausgangsstoffe der Formel (II) sind noch nicht bekannt und als neue Stoffe ebenfalls Gegenstand der vorliegenden Anmeldung.

Sie werden erhalten (Verfahren c), wenn man Carbonsäuren der allgemeinen Formel (V). 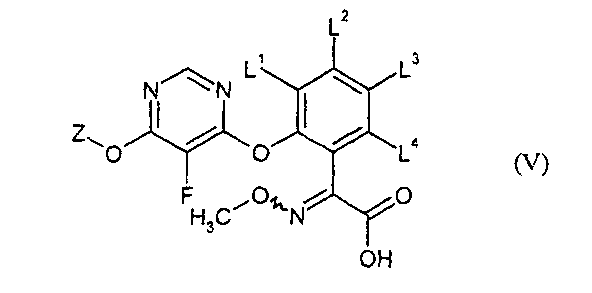 in welcher
- L¹, L², L³, L⁴ und Z: die oben angegebenen Bedeutungen haben,
mit einem Halogenierungsmittel, gegebenenfalls in Gegenwart eines Verdünnungsmittels und gegebenenfalls in Gegenwart eines Katalysators, umsetzt.

Die zur Durchführung des erfindungsgemäßen Verfahrens c) als Ausgangsstoffe benötigten Carbonsäuren sind durch die Formel (V) allgemein definiert. In dieser Formel (V) haben L¹, L², L³, L⁴ und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für L¹, L², L³, L⁴ und Z angegeben wurden.

Die Ausgangsstoffe der Formel (V) sind noch nicht bekannt und als neue Stoffe ebenfalls Gegenstand der vorliegenden Anmeldung.

Sie werden erhalten (Verfahren d), wenn man Carbonsäureester der allgemeinen Formel (VI), 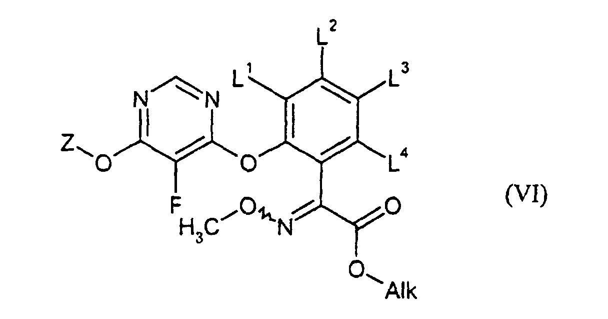 in welcher
- L¹, L², L³, L⁴ und Z: die oben angegebenen Bedeutungen haben und
- Alk: für Alkyl steht,
mit einer Säure, gegebenenfalls in Gegenwart eines Verdünnungsmittels und gegebenenfalls in Gegenwart eines Katalysators, umsetzt.

Die zur Durchführung des erfindungsgemäßen Verfahrens d) als Ausgangsstoffe benötigten Carbonsäureester sind durch die Formel (VI) allgemein definiert. In dieser Formel (VI) haben L¹, L², L³, L⁴ und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für L¹, L², L³, L⁴ und Z angegeben wurden. Alk steht für Alkyl, vorzugsweise für Methyl oder Ethyl.

Die Ausgangsstoffe der Formel (VI) sind noch nicht bekannt und als neue Stoffe ebenfalls Gegenstand der vorliegenden Anmeldung.

Die Ausgangsstoffe der Formel (VI) können erhalten werden (Verfahren e), wenn man 2-(2-Hydroxy-phenyl)-2-alkoxyiminoessigester der allgemeinen Formel (VII), 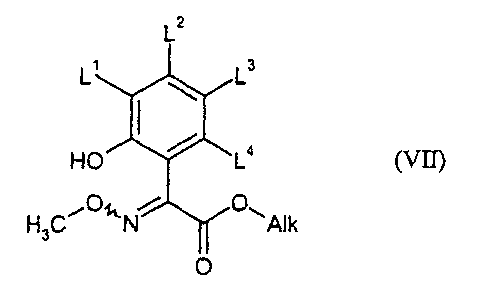 in welcher
- Alk, L¹, L², L³ und L⁴: die oben angegebenen Bedeutungen haben,
mit einem Fluorpyrimidin der allgemeinen Formel (VIII), 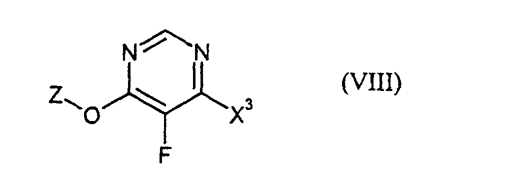 in welcher
- Z: die oben angegebene Bedeutung hat und
- X³: für Halogen steht,
gegebenenfalls in Gegenwart eines Verdünnungsmittels, wie beispielsweise Acetonitril, gegebenenfalls in Gegenwart eines Säureakzeptors, wie beispielsweise Kaliumcarbonat, umsetzt.

Die zur Durchführung des Verfahrens e) als Ausgangsstoffe benötigten 2-(2-Alkoxyphenyl)-2-methoxyiminoessigester sind durch die Formel (VII) allgemein definiert. In dieser Formel (VII) haben Alk, L¹, L², L³ und L⁴ vorzugsweise bzw. insbesondere diejenigen Bedeutungen, die bereits im Zusammenhang mit der Beschreibung der erfindungsgemäßen Verbindungen der Formel (VI) als bevorzugt bzw. als insbesondere bevorzugt für Alk, L¹, L², L³ und L⁴ angegeben wurden.

Die Ausgangsstoffe der Formel (VII) sind bekannt und können nach bekannten Verfahren hergestellt werden (vergleiche z. B. WO-A 94-05626, GB-A 2249092)

Die zur Durchführung des Verfahrens e) weiterhin als Ausgangsstoffe benötigten Fluorpyrimidine sind durch die Formel (VIII) allgemein definiert. In dieser Formel (VIII) hat Z vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für Z angegeben wurde. X³ steht für Halogen, vorzugsweise für Fluor oder Chlor.

Die Ausgangsstoffe der Formel (VIII) sind bekannt und/oder können nach bekannten Methoden hergestellt werden (vergleiche z. B. DE 19737723; Chem.Ber., 90 <1957> 942, 951).

Als Halogenierungsmittel zur Durchführung des erfindungsgemäßen Verfahrens c) kommen alle Reagenzien infrage, die an Kohlenstoff gebundene Hydroxygruppen gegen Halogene austauschen können. Beispielhaft seien genannt: Phosgen, Oxalylchlorid, Phosphortrichlorid, Phosphortribromid, Phosphorpentachlorid, Phosphoroxychlorid, Thionylchlorid oder Thionylbromid. Die Halogenierungsmittel sind bekannte Synthesechemikalien.

Die zur Durchführung des erfindungsgemäßen Verfahrens a) weiterhin als Ausgangsstoffe benötigten Hydroxylaminderivate sind durch die Formel (III) allgemein definiert. In dieser Formel (III) haben R¹ und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R¹ und R² angegeben wurden.

Die Ausgangsstoffe der Formel (III) sind allgemein übliche Synthesechemikalien.

Die zur Durchführung des erfindungsgemäßen Verfahrens b) als Ausgangsstoffe benötigten Hydroxamsäurederivate der allgemeinen Formel (I) mit R² in seiner Bedeutung als Wasserstoff sind erfindungsgemäße Verbindungen und können nach Verfahren a) hergestellt werden.

Die zur Durchführung des erfindungsgemäßen Verfahrens a) weiterhin als Ausgangsstoffe benötigten Halogenverbindungen sind durch die Formel (IV) allgemein definiert. In dieser Formel (IV) hat R² vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R² angegeben wurde. X² steht für Halogen, vorzugsweise für Chlor.

Die Ausgangsstoffe der Formel (IV) sind allgemein übliche Synthesechemikalien.

Als Verdünnungsmittel zur Durchführung der erfindungsgemäßen Verfahren a) und b) kommen alle inerten organischen Lösungsmittel in Betracht. Hierzu gehören beispielhaft und vorzugsweise Ether, wie Diethylether, Diisopropylether, Methyl-tbutylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; oder Sulfone, wie Sulfolan.

Die erfindungsgemäßen Verfahren a) und b) werden gegebenenfalls in Gegenwart eines geeigneten Säureakzeptors durchgeführt. Als solche kommen alle üblichen anorganischen oder organischen Basen infrage. Hierzu gehören beispielhaft und vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren a) und b) in einem größeren Bereich variiert werden. Im allgemeinen arbeitet man bei Temperaturen von -20°C bis 100°C, vorzugsweise bei Temperaturen von -10°C bis 80°C.

Zur Durchführung des erfindungsgemäßen Verfahrens a) zur Herstellung der Verbindungen der Formel (I) setzt man pro Mol des Carbonsäurederivates der Formel (II) im allgemeinen 0,5 bis 15 Mol, vorzugsweise 0,8 bis 8 Mol substituiertes Hydroxylamindcrivat der Formel (III) ein.

Zur Durchführung des erfindungsgemäßen Verfahrens b) zur Herstellung der Verbindungen der Formel (I) setzt man pro Mol des Hydroxamsäurederivates der Formel (I) mit R² in seiner Bedeutung als Wasserstoff im allgemeinen 0,5 bis 15 Mol, vorzugsweise 0.8 bis 8 Mol einer Halogenverbindung der allgemeinen Formel (IV) ein.

Als Verdünnungsmittel zur Durchführung des erfindungsgemäßen Verfahrens c) kommen alle inerten organischen Lösungsmittel in Betracht. Hierzu gehören beispielhaft und vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin oder halogenierte Kohlenwasserstoffe, wie beispielsweise Chlorbenzol, Dichlorbenzol, Dichlormethan, Chloroform, Tetrachlormethan, Dichlorethan oder Trichlorethan.

Als Katalysatoren zur Durchführung des erfindungsgemäßen Verfahrens c) kommen beispielsweise Pyridin oder Dimethylformamid infrage.

Die Reaktionstemperaturen können bei der Durchführung des erfindungsgemäßen Verfahrens c) in einem größeren Bereich variiert werden. Im allgemeinen arbeitet man bei Temperaturen von -10°C bis 100°C, vorzugsweise bei Temperaturen von 0°C bis 80°C.

Zur Durchführung des erfindungsgemäßen Verfahrens c) zur Herstellung der Verbindungen der Formel (II) setzt man pro Mol der Carbonsäure der Formel (V) im allgemeinen 1 bis 15 Mol, vorzugsweise 2 bis 8 Mol Halogenierungsmittel ein.

Als Verdünnungsmittel zur Durchführung des erfindungsgemäßen Verfahrens d) kommen alle inerten organischen Lösungsmittel in Betracht. Hierzu gehören beispielhaft und vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin oder halogenierte Kohlenwasserstoffe, wie beispielsweise Chlorbenzol, Dichlorbenzol, Dichlormethan, Chloroform, Tetrachlormethan, Dichlorethan oder Trichlorethan.

Das erfindungsgemäße Verfahren d) wird gegebenenfalls in Gegenwart einer Säure durchgerührt. Als solche kommen alle anorganischen und organischen Protonen- wie beispielhaft und vorzugsweise auch Lewissäuren, sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen-III-chlorid, Antimonpentachlorid, saure Ionenaustauscher, saure Tonerden und saures Kieselgel.

Die Reaktionstemperaturen können bei der Durchführung des erfindungsgemäßen Verfahrens in einem größeren Bereich variiert werden. Im allgemeinen arbeitet man bei Temperaturen von -50°C bis 80°C, vorzugsweise bei Temperaturen von -20°C bis 50°C.

Zur Durchführung des erfindungsgemäßen Verfahrens d) zur Herstellung der Verbindungen der Formel (V) setzt man pro Mol der Carbonsäureester der Formel (VI) im allgemeinen 1 bis 15 Mol, vorzugsweise 2 bis 8 Mol Säure ein.

Alle erfindungsgemäßen Verfahren werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach allgemein üblichen Verfahren (vergleiche auch die Herstellungsbeispiele).

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Altemaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Dabei lassen sich die erfindungsgemäßen Wirkstoffe mit besonders gutem Erfolg zur Bekämpfung von Getreidekrankheiten, wie beispielsweise gegen Erysiphe-Arten oder Puccinia-Arten, von Krankheiten im Wein-, Obst- und Gemüseanbau, wie beispielsweise gegen Venturia-, Sphaerotheca-, Phytophtora- und Plasmopara-Arten, oder von Reiskrankheiten, wie beispielsweise gegen Pyricularia-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten. Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol,
Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1 -Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-α-methyl- 1H- 1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-( 1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(-4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2.6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl)-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-((3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)- 1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5, 6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(-3'H)-isobenzofuran]-3'-on,

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Bioperrnethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kempolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethzin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Theta-cypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl)tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino)-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon
Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-ylester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N''-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) wie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben; Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die nachfolgenden Beispiele dienen zur Erläuterung der Erfindung. Die Erfindung ist jedoch nicht auf die Beispiele limitiert.

### Herstellungsbeispiele:

### Beispiel 1

### 2-(2-{[6-(2-Chlorphenoxy)-5-fluor-4-pyrimidinyl]oxy}phenyl)-N-hydroxy-2-methoxyimino)-N-methylacetamid

Zu einer Mischung aus 32,45 g (0,39 Mol) N-Methylhydroxylamin Hydrochlorid in 400 ml Tetrahydrofuran gibt man zuerst 47,2 g (0,47 Mol) Triethylamin, kühlt auf 0°C und tropft dann bei dieser Temperatur innerhalb einer Stunde eine Lösung aus 33,6 g (0,078 Mol) 2-{2-[6-(2-Chlorphenoxy)-5-fluor-pyrimidin-4-yloxy]-phenyl}-2-methoxyiminoessigsäurechlorid in 50 ml Tetrahydrofuran zu. Es wird ohne weitere Kühlung noch 18 Stunden gerührt. Das Reaktionsgemisch wird auf 21 Wasser gegossen und drei mal mit jeweils 400 ml Essigsäureethylester extrahiert. Die vereinigten organischen Phasen werden über Natriumsulfat getrocknet und bei vermindertem Druck eingeengt. Der Rückstand wird mit Diisopropylether verrührt, abgesaugt und getrocknet. Man erhält 13,2 g (38 % der Theorie) 2-(2-{[6-(2-Chlorphenoxy)-5-fluor-4-pyrimidinyl]oxy}phenyl)-N-hydroxy-2-(methoxyimino)-N-methylacetamid.
HPLC: logP = 2,82

### Beispiel 2

### 2-(2-{[6-(2-Chlorphenoxy)-5-fluor-4-pyrimidinyl]oxy}phenyl)-N-hydroxy-2-(methoxyimino)-acetamid

Zu einer Mischung aus 0,8 g (0,012 Mol) Hydroxylamin Hydrochlorid in 20 ml Tetrahydrofuran gibt man zuerst 1,4 g (0,014 Mol) Triethylamin, kühlt auf 0°C und tropft dann bei dieser Temperatur eine Lösung aus 1 g (0,0023 Mol) 2-{2-[6-(2-Chlorphenoxy)-5-fluor-pyrimidin-4-yloxy]-phenyl}-2-methoxyiminoessigsäurechlorid in 10 ml Tetrahydrofuran zu. Es wird ohne weitere Kühlung noch 18 Stunden gerührt. Das Reaktionsgemisch wird auf 100 ml 1N Salzsäure gegossen und drei mal mit jeweils 100 ml Essigsäureethylester extrahiert. Die vereinigten organischen Phasen werden über Natriumsulfat getrocknet und bei vermindertem Druck eingeengt.
Der Rückstand wird mit Cyclohexan/Essigester (4:1) an Kieselgel chromatografiert. Man erhält 0,3 g (30 % der Theorie) 2-(2-{[6-(2-Chlorphenoxy)-5-fluor-4-pyrimidinyl]oxy}phenyl)-N-hydroxy-2-(methoxyimino)-acetamid.
HPLC: logP = 2,56

### Beispiel 3

### N-(Acetyloxy)-2-(2-{[6-(2-chlorphenoxy)-5-fluor-4-pyrimidinyl]oxy}phenyl)-2-(methoxyimino)-N-methylacetamid

Zu einer Mischung aus 0,4 g (0,0009 Mol) 2-(2-{[6-(2-Chlorphenoxy)-5-fluor-4-pyrimidinyl]oxy}phenyl)-N-hydroxy-2-(methoxyimino)-N-methylacetamid in 20 ml Tetrahydrofuran gibt man zuerst 0,12 g (0,0012 Mol) Triethylamin, und dann 0,08 g (0,00099 Mol) Acetylchlorid zu und rührt 1 Stunde bei Raumtemperatur. Die Reaktionsmischung wird auf 100 ml Wasser gegossen und 3 mal mit jeweils 50 ml Essigsäureethylester extrahiert. Die vereinigten organischen Phasen werden über Magnesiumsulfat getrocknet und bei vermindertem Druck eingeengt. Der Rückstand wird mit Cyclohexan/Essigester (4:1) an Kieselgel chromatografiert. Man erhält 0,18 g (41,1 % der Theorie) N-(Acetyloxy)-2-(2-{[6-(2-chlorphenoxy)-5-fluor-4-pyrimidinyl]oxy}phenyl)-2-(methoxyimino)-N-methylacetamid.
HPLC: logP = 3,52

Analog den Beispielen 1 bis 3, sowie entsprechend den Angaben in den allgemeinen Verfahrensbeschreibungen a) und b), werden auch die in der nachstehenden Tabelle 1 genannten Verbindungen der Formel (Ia) erhalten.

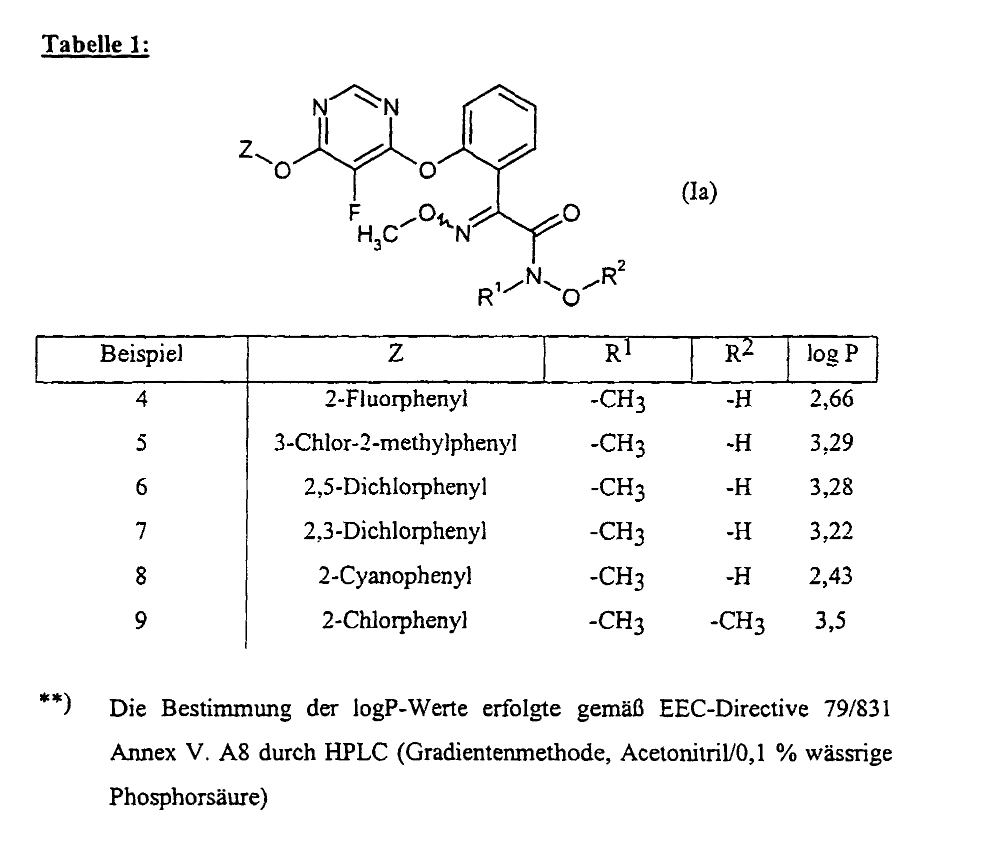

### Herstellung der Vorprodukte

### Beispiel (II-1)

### 2-{2-[6-(2-Chlorphenoxy)-5-fluor-pyrimidin-4-yloxy]-phenyl}-2-methoxyiminoessigsäurechlorid

Zu einer Lösung von 10 g (0,024 Mol) 2-{2-[6-(2-Chlorphemoxy)-5-fluor-pyrimidin-4-yloxyl-phenyl}-2-methoxyiminoessigsäure in 200 ml Dichlormethan gibt man zuerst 50 mg Dimethylformamid und tropft anschließend 3,95 g (0,031 Mol) Oxalylchlorid zu. Es wird es wird 5 Stunden bei 40 °C und dann ohne weitere Wärmezufuhr noch 18 Stunden gerührt. Das Reaktionsgemisch wird bei vermindertem Druck eingeengt. Man erhält 10,4 g (99,6 % der Theorie) 2-{2-[6-(2-Chlorphenoxy)-5-fluor-pyrimidin-4-yloxy]-phenyl}-2-methoxyiminoessigsäurechlorid.

### Beispiel (V-1)

### 2-{2-(6-(2-Chlorphenoxy)-5-fluor-pyrimidin-4-yloxy]-phenyl}-2-methoxyiminoessigsäure

Zu einer Lösung von 4 g (0,0093 Mol) 2-{2-[6-(2-Chlorphenoxy)-5-fluor-pyrimidin-4-yloxy]-phenyl}-2-methoxyiminoessigsäuremethylester in 40 ml Dichlormethan tropft man bei -10°C 46,4 ml (0,046 Mol) Bortribromid zu, erwärmt innerhalb einer Stunde auf Raumtemperatur und rührt noch weitere 2 Stunden. Unter Kühlung werden 300 ml Wasser zugetropft, anschließend die organische Phase abgetrennt. Die wässrige Phase wird noch zweimal mit jeweils 100 ml Dichlormethan extrahiert. Die vereinigten organischen Phasen werden über Kieselgur filtriert, über Magnesiumsulfat getrocknet und bei vermindertem Druck eingeengt. Man erhält 3,8 g (98,3 % der Theorie) 2-{2-[6-(2-Chlorphenoxy)-5-fluor-pyrimidin-4-yloxy]-phenyl} -2-methoxyiminoessigsäure.
NMR (DMSO, TMS): δ = 3,83 (s), 7,34 - 7,67 (m), 8,13 (s), 13,38 (s)

### Beispiel (VI-1)

### 2-{2-[6-(2-Chlorphenoxy)-5-fluor-pyrimidin-4-yloxy]-phenyl}-2-methoximinoessigsäuremethylester

Zu einer Lösung aus 50 g (0,24 Mol) 2-(2-Hydroxyphenyl)-2-methoxyiminoessigsäuremethylester in 600 ml Acetonitril gibt man 40 g (0,29 Mol) gemahlenes Kaliumcarbonat und 58 g (0,24 Mol) 4-(2-Chlorphenoxy)-5,6-difluorpyrimidin und rührt 18 Stunden bei 25°C. Man gießt das Reaktionsgemisch auf 3 l Eiswasser und saugt den entstandenen Feststoff ab. Man erhält 102 g (98,7 % der Theorie) 2-{2-[6-(2-Chlorphenoxy)-5-fluor-pyrimidin-4-yloxy]-phenyl}-2-methoxyiminoessigsäuremethylester.
HPLC: logP = 3,61

### Anwendungsbeispiele

### Beispiel A

### Puccinia-Test (Weizen) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (1), (4), (8) aufgeführten erfindungsgemäßen Stoffe bei einer Aufwandmenge von 125 g/ha einen Wirkungsgrad von 95 % oder mehr.

### Beispiel B

### Phytophthora-Test (Tomate) / protektiv

| | |
|---|---|
| Lösungsmittel | 24,5 Gewichtsteile Aceton |
| | 24,5 Gewichtsteile Dimethylacetamid |
| Emulgator | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Phytophthora infestans inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (1), (5), (7) aufgeführten erfindungsgemäßen Stoffe bei einer Aufwandmenge von 100 g/ha einen Wirkungsgrad von 86 % oder mehr.

### Beispiel C

### Plasmopara-Test (Rebe) / protektiv

| | |
|---|---|
| Lösungsmittel | 24,5 Gewichtsteile Aceton |
| | 24,5 Gewichtsteile Dimethylacetamid |
| Emulgator | 1,0 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 5 Tage im Gewächshaus bei ca. 21°C und ca. 90 % relativer Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (1), (5), (7) aufgeführten erfindungsgemäßen Stoffe bei einer Aufwandmenge von 100 g/ha einen Wirkungsgrad von 95 % oder mehr.

### Beispiel D

### Sphaerotheca-Test (Gurke) / protektiv

| | |
|---|---|
| Lösungsmittel | 24,5 Gewichtsteile Aceton |
| | 24,5 Gewichtteile Dimethylacetamid |
| Emulgator | 1,0 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von Sphaerotheca fuliginea inokuliert. Die Pflanzen werden dann bei ca. 23°C und einer relativen Luftfeuchtigkeit von ca. 70 % im Gewächshaus aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100% bedeutet, dass kein Befall beobachtet wird.

Bei diesem Test zeigen die in den Beispielen (1), (5), (7) aufgeführten erfindungsgemäßen Stoffe bei einer Aufwandmenge von 100 g/ha einen Wirkungsgrad von 95 % oder mehr.

### Beispiel E

### Venturia-Test (Apfel) / protektiv

| | |
|---|---|
| Lösungsmittel | 24,5 Gewichtsteile Aceton |
| | 24,5 Gewichtsteile Dimethylacetamid |
| Emulgator | 1,0 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0% ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (1), (5), (7) aufgeführten erfindungsgemäßen Stoffe bei einer Aufwandmenge 10 von g/ha einen Wirkungsgrad von 96 % oder mehr.

### Beispiel F

### Pyricularia-Test (Reis) / protektiv

| | | |
|---|---|---|
| Lösungsmittel | 25 Gewichtsteile | N,N-Dimethylacetamid |
| Emulgator | 0,6 Gewichtsteile | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Reispflanzen mit der Wirkstoffzubereitung in der angegebenen Aufwandmenge. Nach dem Antrocknen des Spritzbelages werden die Pflanzen mit einer wässrigen Sporensuspension von *Pyricularia oryzae* inokuliert und verbleiben dann 24h bei 100% rel. Luftfeuchte und 26°C. Anschließend werden die Pflanzen in einem Gewächshaus bei 80 % rel. Luftfeuchtigkeit und einer Temperatur von 26°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (1), (4), (5), (7) aufgeführten erfindungsgemäßen Stoffe bei einer Aufwandmenge von 250 g/ha einen Wirkungsgrad von 90 % oder mehr.

### Beispiel G

### Erysiphe-Test (Gerste) / protektiv

| | | |
|---|---|---|
| Lösungsmittel | 50 Gewichtsteile | N, N - Dimethylformamid |
| Emulgator | 1,2 Gewichtsteile | Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und Emulgator auf die gewünschte Konzentration.

Zur Prüfung auf protektive Wirksamkeit bespritzt man junge Getreidepflanzen mit der Wirkstoffzubereitung in der angegebenen Aufwandmenge. 1 Tag nach der Behandlung werden die Pflanzen mit Sporen von Erysiphe graminis f. sp. hordei inokuliert. Anschließend werden die Pflanzen in einem Gewächshaus bei 70 % relativer Luftfeuchtigkeit und einer Temperatur von 18°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen die in den Beispielen (1), (5), (7) aufgeführten erfindungsgemäßen Stoffe bei einer Aufwandmenge von 750 g/ha einen Wirkungsgrad von 80 % oder mehr.

## Patentansprüche

1. Verbindungen der allgemeinen Formel (I), 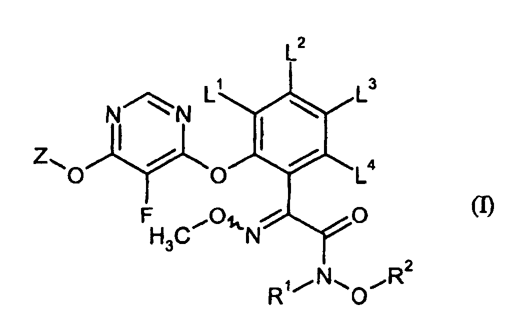 in welcher
Z für jeweils gegebenenfalls einfach bis zweifach durch Halogen, Alkyl, oder Hydroxy substituiertes Cycloalkyl mit 3 bis 7 Kohlenstoffatomen;
für gegebenenfalls durch Alkyl mit 1 bis 4 Kohlenstoffatomen substituiertes Heterocyclyl mit 3 bis 7 Ringgliedern;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino,
Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 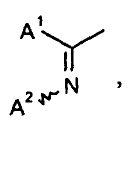 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohenstoffatomen steht,
sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen,
L¹, L², L³ und L⁴ gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen,
R¹ für Wasserstoff oder Alkyl steht und
R² für Wasserstoff, Alkyl, Alkylcarbonyl, Alkoxycarbonyl oder Arylcarbonyl steht.

2. Verbindungen der Formel (I) gemäß Anspruch 1, **dadurch gekennzeichnet, dass**
Z für jeweils gegebenenfalls einfach bis zweifach durch Fluor, Chlor, Methyl, Ethyl oder Hydroxy substituiertes Cyclopentyl oder Cyclohexyl;
für gegebenenfalls durch Methyl oder Ethyl substituiertes Thienyl, Pyridyl oder Furyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl,
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), Hydroxymethyl, Hydroxyethyl, 3-Oxobutyl, Methoxymethyl, Dimethoxymethyl,
Methoxy, Ethoxy, n- oder i-Propoxy, Methoxymethyl, Ethoxymethyl,
Methylthio, Ethylthio, n- oder i-Propylthio, Methylsulfinyl, Ethylsulfinyl, Methylsulfonyl oder Ethylsulfonyl, Methylthiomethyl, Ethylthiomethyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy
oder eine Gruppierung 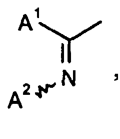 wobei
A¹ für Wasserstoff, Methyl oder Hydroxy steht und
A² für Hydroxy, Methoxy, Ethoxy, Amino, Methylamino, Phenyl, Benzyl oder Hydroxyethyl steht, sowie
jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl,
L¹, L², L³ und L⁴ gleich oder verschieden sind und unabhängig voneinander jeweils für Wasserstoff, Methyl, Ethyl, n- oder i-Propyl, n-, i-, s- oder t-Butyl stehen,
R¹ für Wasserstoff oder Alkyl steht,
R² für Wasserstoff, Alkyl, Alkylcarbonyl, Alkoxycarbonyl oder Arylcarbonyl steht.

3. Verbindungen der Formel (I) gemäß Anspruch 1, **dadurch gekennzeichnet, dass**
Z für jeweils gegebenenfalls einfach bis dreifach, gleich oder verschieden substituiertes Phenyl steht, wobei die Substituenten aus der nachstehenden Aufzählung ausgewählt sind:
Fluor, Chlor, C₁-C₄-Alkyl oder Cyano,
L¹, L², L³ und L⁴ für Wasserstoff stehen,
R¹ für C₁-C₄-Alkyl steht, und
R² für Wasserstoff oder Methyl steht.

4. Verbindungen der allgemeinen Formel (II), 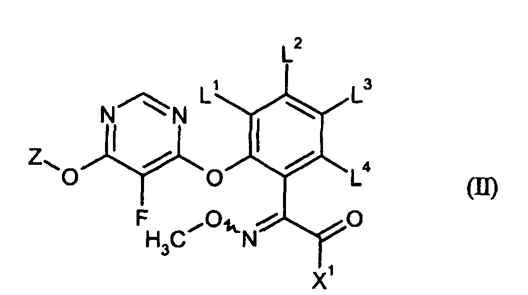 in welcher
Z, L¹, L², L³ und L⁴ die in Anspruch 1 angegebene Bedeutung haben und
X¹ für Halogen steht.

5. Verbindungen der allgemeinen Formel (V), 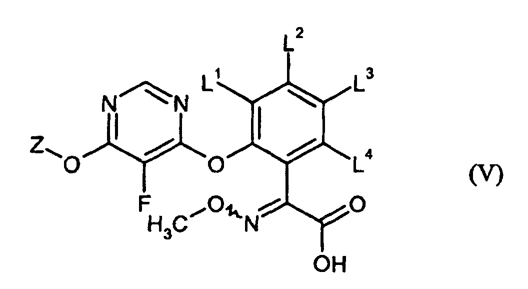 in welcher
L¹, L², L³, L⁴ und Z die in Anspruch 1 angegebenen Bedeutungen haben.

6. Verfahren zur Herstellung von Verbindungen der allgemeinen Formel (I), wie in Anspruch 1 definiert, **dadurch gekennzeichnet, dass** man
a) Carbonsäurederivate der Formel (II), 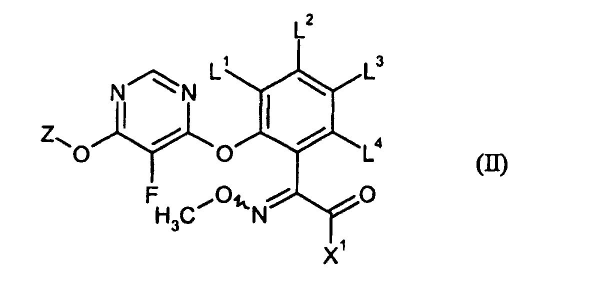 in welcher
L¹, L², L³, L⁴ und Z die in Anspruch 1 angegebenen Bedeutungen haben und
X¹ für Halogen steht,
mit einem substituierten oder unsubstituierten Hydroxylaminderivat der allgemeinen Formel (III),
in welcher 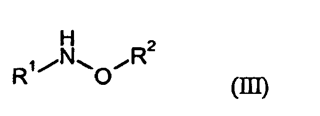
R¹ und R² die in Anspruch 1 angegebenen Bedeutungen haben,
gegebenenfalls in Gegenwart eines Verdünnungsmittels und gegebenenfalls in Gegenwart eines Säureakzeptors, umsetzt, oder wenn man
b) Verbindungen der allgemeinen Formel (I) mit R² in seiner Bedeutung als Wasserstoff mit einer Halogenverbindung der allgemeinen Formel (IV),
X²-R^{2'} (IV)
in welcher
R^{2'} für Alkyl, Alkylcarbonyl, Alkoxycarbonyl oder Arylcarbonyl steht und
X² für Halogen steht,
gegebenenfalls in Gegenwart eines Verdünnungsmittels, gegebenenfalls in Gegenwart eines Säureakzeptors, umsetzt.

7. Mittel enthaltend Streckmittel und/oder Trägerstoffe sowie gegebenenfalls oberflächenaktive Stoffe, **gekennzeichnet durch** einen Gehalt an mindestens einer Verbindung wie in den Ansprüchen 1 bis 3 definiert.

8. Verfahren zur Bekämpfung von Schädlingen, **dadurch gekennzeichnet, dass** man Verbindungen wie in den Ansprüchen 1 bis 3 bzw. Mittel wie in Anspruch 7 definiert auf Schädlinge und/oder ihren Lebensraum einwirken lässt.

9. Verfahren zur Herstellung von Schädlingsbekämpfungsmitteln, **dadurch gekennzeichnet, dass** man Verbindungen der Formel (I) wie in den Ansprüchen 1 bis 3 definiert mit Streckmitteln und/oder oberflächenaktiven Mitteln vermischt.

10. Verwendung von Verbindungen wie in den Ansprüchen 1 bis 3 bzw. von Mitteln wie in Anspruch 7 definiert zur Bekämpfung von Schädlingen.

11. Verwendung von Verbindungen der Formel (II) wie in Anspruch 4 definiert und von Verbindungen der Formel (V) wie in Anspruch 5 definiert als Zwischenprodukte.

## Claims

1. Compounds of the general formula (I) 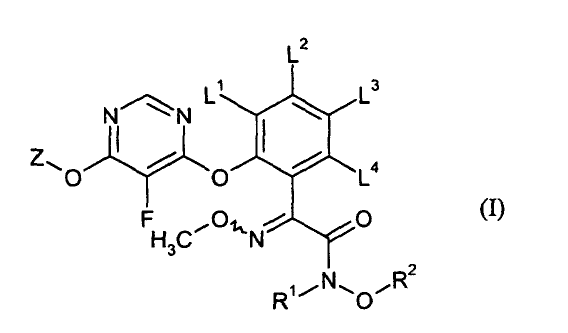 in which
Z represents cycloalkyl having 3 to 7 carbon atoms which is optionally mono- to disubstituted by halogen, alkyl or hydroxyl;
represents heterocyclyl having 3 to 7 ring members which is optionally substituted by alkyl having 1 to 4 carbon atoms;
or represents phenyl or naphthyl, each of which is optionally mono-to tetrasubstituted by identical or different substituents, where the possible substituents are preferably selected from the list below:
halogen, cyano, nitro, amino, hydroxyl, formyl, carboxyl, carbamoyl, thiocarbamoyl;
in each case straight-chain or branched alkyl, hydroxyalkyl, oxoalkyl, alkoxy, alkoxyalkyl, alkylthioalkyl, dialkoxyalkyl, alkylthio, alkylsulphinyl or alkylsulphonyl having in each case 1 to 8 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 11 identical or different halogen atoms;
in each case straight-chain or branched alkylamino, dialkylamino,
alkylcarbonyl, alkylcarbonyloxy, alkoxycarbonyl, alkylaminocarbonyl, dialkylaminocarbonyl, arylalkylaminocarbonyl, dialkylaminocarbonyloxy, alkenylcarbonyl or alkinylcarbonyl, having 1 to 6 carbon atoms in the hydrocarbon chains in question;
cycloalkyl or cycloalkyloxy having in each case 3 to 6 carbon atoms;
in each case doubly attached alkylene having 3 to 4 carbon atoms, oxyalkylene having 2 or 3 carbon atoms or dioxyalkylene having 1 or 2 carbon atoms, each of which radicals is optionally mono- to tetrasubstituted by identical or different radicals from the group consisting of fluorine, chlorine, oxo, methyl, trifluoromethyl and ethyl;
or a grouping 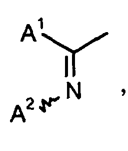 in which
A¹ represents hydrogen, hydroxyl or alkyl having 1 to 4 carbon atoms or cycloalkyl having 1 to 6 carbon atoms and
A² represents hydroxyl, amino, methylamino, phenyl, benzyl or represents in each case optionally cyano-, hydroxyl-, alkoxy-, alkylthio-, alkylamino-, dialkylamino- or phenyl-substituted alkyl or alkoxy having 1 to 4 carbon atoms, or represents alkenyloxy or alkinyloxy having in each case 2 to 4 carbon atoms,
and also phenyl, phenoxy, phenylthio, benzoyl, benzoylethenyl, cinnamoyl, heterocyclyl or phenylalkyl, phenylalkyloxy, phenylalkylthio, or heterocyclylalkyl, having in each case 1 to 3 carbon atoms in the alkyl moieties in question, each of which radicals is optionally mono- to trisubstituted in the cyclic moiety by halogen and/or straight-chain or branched alkyl or alkoxy having I to 4 carbon atoms,
L¹, L², L³ and L⁴ are identical or different and independently of one another each represent hydrogen, halogen, cyano, nitro, or alkyl, alkoxy, alkylthio, alkylsulphinyl or alkylsulphonyl having in each case 1 to 6 carbon atoms, each of which radicals is optionally substituted by 1 to 5 halogen atoms,
R¹ represents hydrogen or alkyl and
R² represents hydrogen, alkyl, alkylcarbonyl, alkoxycarbonyl or arylcarbonyl.

2. Compounds of the formula (I) according to Claim 1, **characterized in that**
Z represents cyclopentyl or cyclohexyl, each of which is optionally mono- to disubstituted by fluorine, chlorine, methyl, ethyl or hydroxyl;
represents furyl, pyridyl or thienyl which is optionally substituted by methyl or ethyl;
or represents phenyl or naphthyl, each of which is optionally mono-to tetrasubstituted by identical or different substituents, where the possible substituents are preferably selected from the list below:
fluorine, chlorine, bromine, iodine, cyano, nitro, amino, hydroxyl, formyl, carboxyl, carbamoyl, thiocarbamoyl,
methyl, ethyl, n- or i-propyl, n-, i-, s- or t-butyl, 1-, 2-, 3-, neopentyl, 1-, 2-, 3-, 4-(2-methylbutyl), 1-, 2-, 3-hexyl, 1-, 2-, 3-, 4-, 5-(2-methylpentyl), 1-, 2-, 3-(3-methylpentyl), 2-ethylbutyl, 1-, 3-, 4-(2,2-dimetylbutyl), 1-, 2-(2,3-dimethylbutyl), hydroxymethyl, hydroxyethyl, 3-oxobutyl, methoxymethyl, dimethoxymethyl,
methoxy, ethoxy, n- or i-propoxy, methoxymethyl, ethoxymethyl,
methylthio, ethylthio, n- or i-propylthio, methylsulphinyl, ethylsulphinyl, methylsulphonyl or ethylsulphonyl, methylthiomethyl, ethylthiomethyl,
vinyl, allyl, 2-methylallyl, propen-1-yl, crotonyl, propargyl, vinyloxy, allyloxy, 2-methylallyloxy, propen-1-yloxy, crotonyloxy, propargyloxy;
trifluoromethyl, trifluoroethyl,
difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl,
methylamino, ethylamino, n- or i-propylamino, dimethylamino, diethylamino,
acetyl, propionyl, methoxycarbonyl, ethoxycarbonyl, methylaminocarbonyl, ethylaminocarbonyl, dimethylaminocarbonyl, diethylaminocarbonyl, dimethylaminocarbonyloxy, diethylaminocarbonyloxy, benzylaminocarbonyl, acryloyl, propioloyl,
cyclopentyl, cyclohexyl,
in each case doubly attached propanediyl, ethyleneoxy, methylenedioxy, ethylenedioxy, each of which is optionally mono-to tetrasubstituted by identical or different substituents from the group consisting of fluorine, chlorine, oxo, methyl and trifluoromethyl
or a grouping 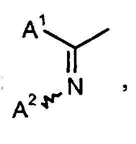 where
A¹ represents hydrogen, methyl or hydroxyl and
A² represents hydroxyl, methoxy, ethoxy, amino, methylamino, phenyl, benzyl or hydroxyethyl, and also
phenyl, phenoxy, phenylthio, benzoyl, benzoylethenyl, cinnamoyl, benzyl, phenylethyl, phenylpropyl, benzyloxy, benzylthio, 5,6-dihydro-1,4,2-dioxazin-3-ylmethyl, triazolylmethyl, benzoxazol-2-ylmethyl, 1,3-dioxan-2-yl, benzimidazol-2-yl, dioxol-2-yl, oxadiazolyl, each of which is optionally mono- to trisubstituted in the cyclic moiety by halogen and/or straight-chain or branched alkyl or alkoxy having 1 to 4 carbon atoms,
L¹, L², L³ and L⁴ are identical or different and independently of one another each represents hydrogen, methyl, ethyl, n- or i-propyl, n-, i-, s- or t-butyl,
R¹ represents hydrogen or alkyl,
R² represents hydrogen, alkyl, alkylcarbonyl, alkoxycarbonyl or arylcarbonyl.

3. Compounds of the formula (I) according to Claim 1, **characterized in that**
Z represents phenyl which is in each case optionally mono- to trisubstituted by identical or different substituents, where the substituents are selected from the list below:
fluorine, chlorine, C₁-C₄-alkyl or cyano,
L¹, L², L³ and L⁴ represent hydrogen,
R¹ represents C₁-C₄-alkyl, and
R² represents hydrogen or methyl.

4. Compounds of the general formula (II) 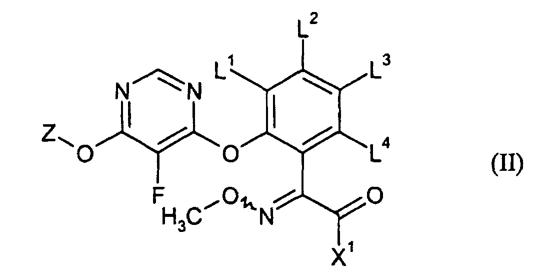 in which
Z, L¹, L², L³ and L⁴ are as defined in claim 1 and
X¹ represents halogen.

5. Compounds of the general formula (V) 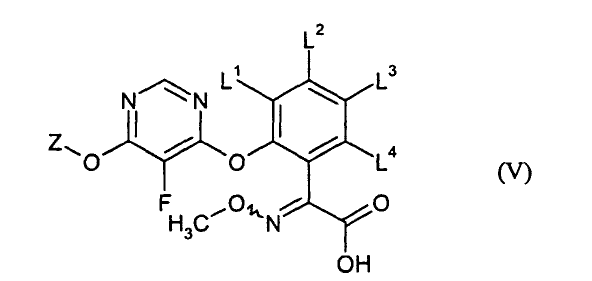 in which
L¹, L², L³, L⁴ and Z are as defined in claim 1.

6. Process for preparing compounds of the general formula (I), as defined in Claim 1, **characterized in that**
a) carboxylic acid derivatives of the formula (II) 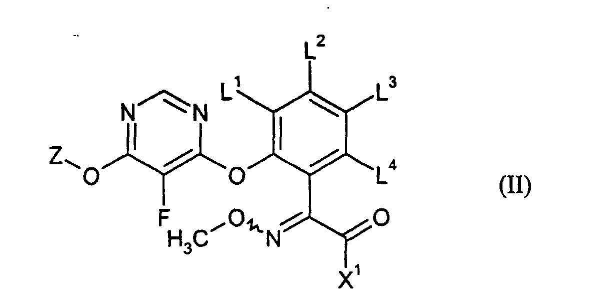 in which
L¹, L², L³, L⁴ and Z are as defined in claim 1 and
X¹ represents halogen,
are reacted with a substituted or unsubstituted hydroxylamine derivative of the general formula (III) 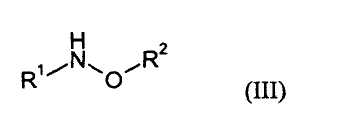 in which
R¹ and R² are as defined in claim 1,
if appropriate in the presence of a diluent and if appropriate in the presence of an acid acceptor, or when
b) compounds of the general formula (I) where R² is hydrogen are reacted with a halogen compound of the general formula (IV)
X²-R^{2'} (IV)
in which
R^{2'} represents alkyl, alkylcarbonyl, alkoxycarbonyl or arylcarbonyl and
X² represents halogen,
if appropriate in the presence of a diluent, if appropriate in the presence of an acid acceptor.

7. Composition comprising extenders and/or carriers and also, if appropriate, surfactants, **characterized in that** it comprises at least one compound as defined in Claims 1 to 3.

8. Method for controlling pests, **characterized in that** compounds as defined in Claims 1 to 3 or compositions as defined in Claim 7 is allowed to act on pests and/or their habitat.

9. Process for preparing pesticides, **characterized in that** compounds of the formula (I) as defined in Claims 1 to 3 are mixed with extenders and/or surfactants.

10. Use of compounds as defined in Claims 1 to 3 or of compositions as defined in Claim 7 for controlling pests.

11. Use of compounds of the formula (II) as defined in Claim 4 and of compounds of the formula (V) as defined in Claim 5 as intermediates.

## Revendications

1. Composés de formule générale (I), 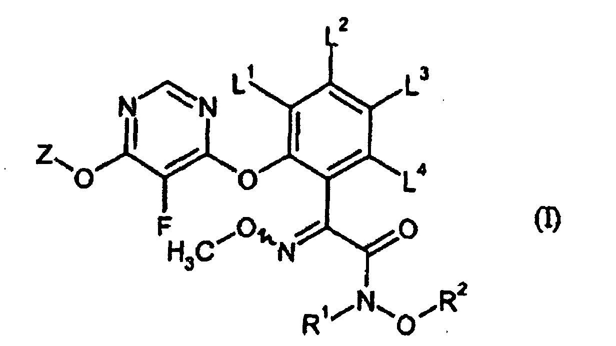 dans laquelle
Z représente un groupe cycloalkyle ayant 3 à 7 atomes de carbone, éventuellement substitué dans chaque cas une ou deux fois par un radical halogéno, alkyle ou hydroxy ;
un reste hétérocyclyle à noyau de 3 à 7 chaînons éventuellement substitué par un radical alkyle ayant 1 à 4 atomes de carbone ;
ou bien un reste phényle ou naphtyle chacun éventuellement substitué une à quatre fois identiques ou différentes, les substituants possibles étant choisis avantageusement parmi ceux énumérés ci-après : halogéno, cyano, nitro, amino, hydroxy, formyle, carboxy, carbamoyle, thiocarbamoyle ;
alkyle, hydroxyalkyle, oxoalkyle, alkoxy, alkoxyalkyle, alkylthioalkyle, dialkoxyalkyle, alkylthio, alkylsulfinyle ou alkylsulfonyle chacun linéaire ou ramifié, ayant chacun 1 à 8 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié, ayant chacun 1 à 6 atomes de carbone et 1 à 13 atomes d'halogènes identiques ou différents ;
halogénalcényle ou halogénalcényloxy chacun linéaire ou ramifié, ayant chacun 2 à 6 atomes de carbone et 1 à 11 atomes d'halogènes identiques ou différents ;
alkylamino, dialkylamino chacun linéaire ou ramifié, alkylcarbonyle, alkylcarbonyloxy, alkoxycarbonyle, alkylaminocarbonyle, dialkylaminocarbonyle, aryl-alkylaminocarbonyle, dialkylaminocarbonyloxy, alcényl-carbonyle ou alcynylcarbonyle ayant 1 à 6 atomes de carbone dans chacune des chaînes hydrocarbonées ;
cycloalkyle ou cycloalkyloxy ayant chacun 3 à 6 atomes de carbone ;
alkylène ayant 3 ou 4 atomes de carbone, oxyalkylène ayant 2 ou 3 atomes de carbone ou dioxyalkylène ayant 1 ou 2 atomes de carbone, comprenant chacun deux liaisons et chacun étant éventuellement substitué une à quatre fois identiques ou différentes par un radical fluoro, chloro, oxo, méthyle, trifluorométhyle ou éthyle ;
ou un groupement 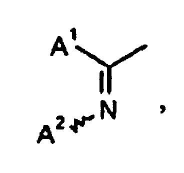 dans lequel
A¹ représente l'hydrogène, un groupe hydroxy ou un reste alkyle ayant 1 à 4 atomes de carbone ou cycloalkyle ayant 1 à 6 atomes de carbone, et
A² est un groupe hydroxy, amino, méthylamino, un reste phényle, benzyle ou un reste alkyle ou alkoxy ayant 1 à 4 atomes de carbone, chacun éventuellement substitué par un radical cyano, hydroxy, alkoxy, alkylthio, alkylamino, dialkylamino ou phényle, ou un reste alcényloxy ou alcynyloxy ayant chacun 2 à 4 atomes de carbone, ainsi qu'un reste phényle, phénoxy, phénylthio, benzoyle, benzoyléthényle, cinnamoyle, hétérocyclyle ou phénylalkyle, phénylalkyloxy, phénylalkylthio ou hétérocyclylalkyle ayant chacun 1 à 3 atomes de carbone dans chacune des parties alkyle, chacun éventuellement substitué dans le noyau une à trois fois par un radical halogéno et/ou par un radical alkyle ou alkoxy linéaire ou ramifié ayant 1 à 4 atomes de carbone,
L¹, L², L³ et L⁴ sont identiques ou différents et représentent, indépendamment les uns des autres, l'hydrogène, un radical halogéno, cyano, nitro, un reste alkyle, alkoxy, alkylthio, alkylsulfinyle ou alkylsulfonyle ayant chacun 1 à 6 atomes de carbone et chacun étant éventuellement substitué par 1 à 5 atomes d'halogènes,
R¹ représente l'hydrogène ou un reste alkyle et
R² représente l'hydrogène, un reste alkyle, alkylcarbonyle, alkoxycarbonyle ou arylcarbonyle.

2. Composés de formule (I) suivant la revendication 1, **caractérisés en ce que**
Z représente un reste cyclopentyle ou cyclohexyle chacun éventuellement substitué une ou deux fois par un radical fluoro, chloro, méthyle, éthyle ou hydroxy ;
un reste thiényle, pyridyle ou furyle éventuellement substitué par un radical méthyle ou éthyle ;
ou bien un reste phényle ou naphtyle chacun éventuellement substitué une à quatre fois identiques ou différentes, les substituants possibles étant avantageusement choisis parmi ceux énumérés ci-après : fluor, chlore, brome, iode, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, 1-, 2-, 3-néopentyle, 1-, 2-, 3-, 4-(2-méthylbutyle), 1-, 2-, 3-hexyle, 1-, 2-, 3-, 4-, 5-(2-méthylpentyle), 1-, 2-, 3-(3-méthylpentyle), 2-éthylbutyle, 1-, 3-, 4-(2,2-diméthylbutyle), 1-, 2-(2,3-diméthylbutyle), hydroxyméthyle, hydroxyéthyle, 3-oxobutyle, méthoxyméthyle, diméthoxyméthyle, méthoxy, éthoxy, n-propoxy, isopropoxy, méthoxyméthyle, éthoxyméthyle,
méthylthio, éthylthio, n-propylthio, isopropylthio, méthylsulfinyle, éthylsulfinyle, méthylsulfonyle, éthylsulfonyle, méthylthiométhyle, éthylthiométhyle, vinyle, allyle, 2-méthylallyle, propène-1-yle, crotonyle, propargyle, vinyloxy, allyloxy, 2-méthylallyloxy, propène-1-yloxy, crotonyloxy, propargyloxy ; trifluorométhyle, trifluoréthyle,
difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle,
méthylamino, éthylamino, n-propylamino, isopropylamino, diméthylamino, diéthylamino,
acétyle, propionyle, méthoxycarbonyle, éthoxycarbonyle, méthylaminocarbonyle, éthylaminocarbonyle, diméthyl-aminocarbonyle, diéthylaminocarbonyle, diméthylamino-carbonyloxy, diéthylaminocarbonyloxy, benzylaminocarbonyle, acryloyle, propioloyle,
cyclopentyle, cyclohexyle,
propanediyle, éthylènoxy, méthylènedioxy, éthylènedioxy comprenant chacun deux liaisons et chacun éventuellement substitué une à quatre fois identiques ou différentes par un radical fluoro, chloro, oxo, méthyle ou trifluorométhyle, ou un groupement 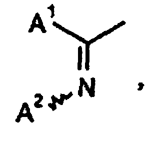 dans lequel
A¹ représente l'hydrogène, un radical méthyle ou hydroxy et
A² est un radical hydroxy, méthoxy, éthoxy, amino, méthylamino, phényle, benzyle ou hydroxyéthyle,
ainsi que
phényle, phénoxy, phénylthio, benzoyle, benzoyléthényle, cinnamoyle, benzyle, phényléthyle, phénylpropyle, benzyloxy, benzylthio, 5,6-dihydro-1,4,2-dioxazine-3-ylméthyle, triazolylméthyle, benzoxazole-2-ylméthyle, 1,3-dioxanne-2-yle, benzimidazole-2-yle, dioxole-2-yle, oxadiazolyle chacun éventuellement substitué dans le noyau une à trois fois par un radical halogéno et/ou un radical alkyle ou alkoxy linéaire ou ramifié ayant 1 à 4 atomes de carbone,
L¹, L², L³ et L⁴ sont identiques ou différents et représentent chacun, indépendamment des autres, l'hydrogène, un radical méthyle, éthyle, n-propyle, isopropyle, n-butyle, isobutyle, sec.-butyle ou tertiobutyle,
R¹ est l'hydrogène ou un reste alkyle,
R² est l'hydrogène, un reste alkyle, alkylcarbonyle, alkoxycarbonyle ou arylcarbonyle.

3. Composés de formule (I) suivant la revendication 1, **caractérisés en ce que**
Z est un reste phényle éventuellement substitué une à trois fois identiques ou différentes, les substituants étant choisis parmi ceux énumérés ci-après : fluor, chlore, alkyle en C₁ à C₄ ou cyano,
L¹, L², L³ et L⁴ représentent l'hydrogène,
R¹ est un reste alkyle en C₁ à C₄ et
R² est l'hydrogène ou un reste méthyle.

4. Composés de formule générale (II), 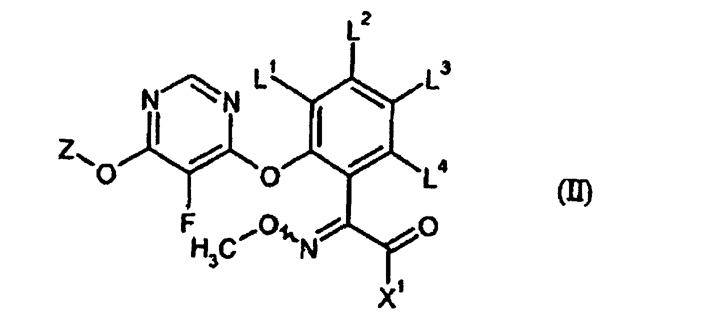 dans laquelle
Z, L¹, L², L³ et L⁴ ont la définition indiquée dans la revendication 1 et
X¹ représente un halogène.

5. Composés de formule générale (V), 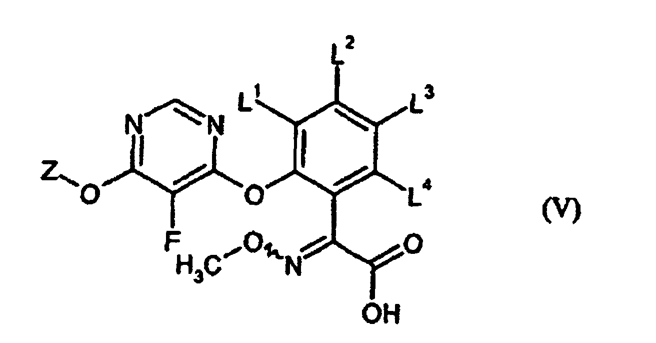 dans laquelle
L¹, L², L³, L⁴ et Z ont les définitions indiquées dans la revendication 1.

6. Procédé de production de composés de formule générale (I) telle que définie dans la revendication 1, **caractérisé en ce que**
a) on fait réagir des dérivés d'acides carboxyliques de formule (II), 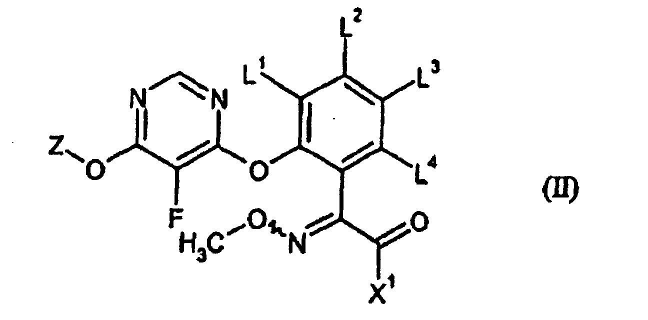 dans laquelle
L¹, L², L³, L⁴ et Z ont les définitions indiquées dans la revendication 1 et
X¹ représente un halogène,
avec un dérivé d'hydroxylamine substitué ou non substitué de formule générale (III), 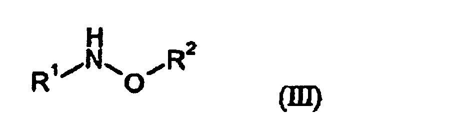 dans laquelle
R¹ et R² ont les définitions indiquées dans la revendication 1,
éventuellement en présence d'un diluant et, le cas échéant, en présence d'un accepteur d'acide, ou bien
b) on fait réagir des composés de formule générale (I) dans laquelle R² est défini comme étant l'hydrogène, avec un composé halogéné de formule générale (IV),
**X**^{**2**}**-R**^{**2'**} **(IV)**
dans laquelle
R^{2'} est un reste alkyle, alkylcarbonyle, alkoxycarbonyle ou arylcarbonyle et
X² représente un halogène,
éventuellement en présence d'un diluant et, le cas échéant, en présence d'un accepteur d'acide.

7. Compositions contenant des diluants et/ou des supports ainsi que, le cas échéant, des agents tensioactifs, **caractérisées par** une teneur en au moins un composé tel que défini dans les revendications 1 à 3.

8. Procédé pour combattre des parasites, **caractérisé en ce qu'**on fait agir des composés tels que définis dans les revendications 1 à 3 ou une composition telle que définie dans la revendication 7 sur les parasites et/ou sur leur milieu.

9. Procédé de préparation de compositions pesticides, **caractérisé en ce qu'**on mélange des composés de formule (I) tels que définis dans les revendications 1 à 3 avec des diluants et/ou des agents tensioactifs.

10. Utilisation de composés tels que définis dans les revendications 1 à 3 ou de compositions telles que définies dans la revendication 7 pour combattre des parasites.

11. Utilisation de composés de formule (II) comme défini dans la revendication 4 et de composés de formule (V) comme défini dans la revendication 5 comme produits intermédiaires.
